# EUROPEAN PATENT SPECIFICATION

(11) **EP 2 425 245 B1**
(45) Date of publication and mention of the grant of the patent: **05.10.2016**
(21) Application number: 10719732.9
(22) Date of filing: 23.04.2010
(51) Int. Cl.: G01N 33/50, G01N 33/53, G01N 33/68, C07K 14/78

(54) **USE OF ENDOSTATIN AS A MARKER OF HEART FAILURE**
Verwendung von Endostatin als Marker bei Herzversagen
Utilisation d'endostatine en tant que marqueur d'une insuffisance cardiaque

(30) Priority: 27.04.2009 EP 09005801
(43) Date of publication of application: 07.03.2012
(73) Proprietor: Roche Diagnostics GmbH, 68305 Mannheim (DE); F.Hoffmann-La Roche AG, 4070 Basel (CH)
(72) Inventor: WIENHUES-THELEN, Ursula-Henrike, 82152 Krailling (DE); BLOCK, Dirk, 83673 Bichl (DE); HUEDIG, Hendrik, 82377 Penzberg (DE)
(74) Representative: Burger, Alexander
(86) International application number: PCT/EP2010/002513
(87) International publication number: WO 2010/124821

(56) References cited:
- EP-A1- 1 967 853
- NASREEN NAJMUNNISA ET AL: "Pleural mesothelial cell (PMC) defense mechanisms against malignancy." ONCOLOGY RESEARCH 2003, vol. 14, no. 3, 2003, pages 155-161, XP008113266 ISSN: 0965-0407
- R&D SYSTEMS, INC.: "Quantikine: Human Endostatin Immunoassay" INTERNET ARTICLE, [Online] 2009, pages 1-16, XP002550191 Retrieved from the Internet: URL:http://www.rndsystems.com/pdf/dnst0.pd f> [retrieved on 2009-10-15]
- ISOBE ET AL: "Ischemia-induced Release of Endostatin from Cardiac Tissues Via Modulation of Ornithine Decarboxylase Activity" JOURNAL OF CARDIAL FAILURE, vol. 12, no. 8, 1 October 2006 (2006-10-01), pages S165-S166, XP005674306 CHURCHILL LIVINGSTONE, NAPERVILLE, IL, US ISSN: 1071-9164
- ISOBE ET AL: "Endogenous endostatin attenuates postinfarction ventricular remodeling by inhibiting matrix metalloproteinase activity" JOURNAL OF MOLECULAR AND CELLULAR CARDIOLOGY, vol. 42, no. 6, 25 May 2007 (2007-05-25), page S151, XP022091344 ACADEMIC PRESS, GB ISSN: 0022-2828
- "Myocardial Ischemia and Infarction" JOURNAL OF THE AMERICAN COLLEGE OF CARDIOLOGY, vol. 53, no. 10, 10 March 2009 (2009-03-10), pages A305-A354, XP026001774 ELSEVIER, NEW YORK, NY, US ISSN: 0735-1097 [retrieved on 2009-03-03] & LAUTEN ALEXANDER ET AL: "Extracellular Matrix Processing Is Activated During Early Postischemic Reperfusion with Differential Effects of Temperature on Matrix Degradation and Endothelial Barrier Function" JOURNAL OF THE AMERICAN COLLEGE OF CARDIOLOGY, vol. 53, no. 10, Suppl. 1, March 2009 (2009-03), page A306, & 58TH ANNUAL SCIENTIFIC SESSION OF THE AMERICAN-COLLEGE-OF-CARDIOLOGY; ORLANDO, FL, USA; MARCH 28 -31, 2009 ISSN: 0735-1097
- PETERSEN JOHN W ET AL: "Inflammatory biomarkers in heart failure" CONGESTIVE HEART FAILURE, vol. 12, no. 6, 1 November 2006 (2006-11-01), pages 324-328, XP002501143 CHF INC., GREENWICH, CT, US ISSN: 1527-5299
- JORTANI S A ET AL: "Strategies for developing biomarkers of heart failure" CLINICAL CHEMISTRY, vol. 50, no. 2, 1 February 2004 (2004-02-01), pages 265-278, XP002355516 AMERICAN ASSOCIATION FOR CLINICAL CHEMISTRY, WASHINGTON, DC ISSN: 0009-9147

## Description

### Field of the Invention

The present invention relates to a method for diagnosing heart failure in an individual comprising the steps of a) measuring in a sample obtained from the individual the concentration of the marker endostatin, of b) optionally measuring in the sample the concentration of one or more other marker(s) of heart failure, wherein said one or more other marker(s) is selected from the group consisting of a natriuretic peptide marker, a cardiac troponin marker, and a marker of inflammation and of c) diagnosing heart failure by comparing the concentration determined in step (a) and optionally the concentration(s) determined in step (b) to the concentration of this marker or these markers as established in a control sample, wherein said sample is selected from the group consisting of serum, plasma, and whole blood. Also disclosed are the use of endostatin as a marker protein in the diagnosis of heart failure.

### Background of the Invention

Heart failure (HF) is a major and growing public health problem. In the United States for example approximately 5 million patients have HF and over 550 000 patients are diagnosed with HF for the first time each year (In: American Heart Association, Heart Disease and Stroke Statistics: 2008 Update, Dallas, Texas, American Heart Association (2008)). Similarly US-statistics show that HF is the primary reason for 12 to 15 million office visits and 6.5 million hospital days each year. From 1990 to 1999, the annual number of hospitalizations has increased from approximately 810 000 to over 1 million for HF as a primary diagnosis and from 2.4 to 3.6 million for HF as a primary or secondary diagnosis. In 2001, nearly 53 000 patients died of HF as a primary cause. Heart failure is primarily a condition of the elderly, and thus the widely recognized "aging of the population" also contributes to the increasing incidence of HF. The incidence of HF approaches 10 per 1000 in the population after age 65. In the US alone, the total estimated direct and indirect costs for HF in 2005 were approximately $27.9 billion and approximately $2.9 billion annually is spent on drugs for the treatment of HF (cf. the above cited AHA-statistics).

### Heart Failure

Heart Failure is characterized by a loss in the heart's ability to pump as much blood as the body needs. Failure does not mean that the heart has stopped pumping but that it is failing to pump blood as effectively as it should.

The NYHA **[New York Heart Association]** and the ACC/AHA **[American Association of Cardiology/American Heart Association]** have both established functional classes of HF to gauge the progression of the disease. The NYHA classification scheme has four classes of disease state: Class 1 is asymptomatic at any level of exertion. Class 2 is symptomatic at heavy exertion and Classes III and IV are symptomatic at light and no exertion, respectively.

In the four stage ACC/AHA scheme, Stage A is asymptomatic but is at risk for developing HF. Stage B there is evidence of cardiac dysfunction without symptoms. In Stage C there is evidence of cardiac dysfunction with symptoms. In Stage D, the subject has symptoms of HF despite maximal therapy.

### Etiology of HF

Medically, heart failure (HF) must be appreciated as being a complex disease. It may be caused by the occurrence of a triggering event such as a myocardial infarction (heart attack) or be secondary to other causes such as hypertension, diabetes or cardiac malformations such as valvular disease. Myocardial infarction or other causes of HF result in an initial decline in the pumping capacity of the heart, for example by damaging the heart muscle. This decline in pumping capacity may not be immediately noticeable, due to the activation of one or more compensatory mechanisms. However, the progression of HF has been found to be independent of the patient's hemodynamic status. Therefore, the damaging changes caused by the disease are present and ongoing even while the patient remains asymptomatic. In fact, the compensatory mechanisms which maintain normal cardiovascular function during the early phases of HF may actually contribute to progression of the disease in the long run, for example by exerting deleterious effects on the heart and its capacity to maintain a sufficient level of blood flow in the circulation.

Some of the more important pathophysiological changes which occur in HF are (i) activation of the hypothalamic-pituitary-adrenal axis, (ii) systemic endothelial dysfunction and (iii) myocardial remodeling.
(i) Therapies specifically directed at counteracting the activation of the hypothalamic-pituitary-adrenal axis include beta-adrenergic blocking agents (B-blockers), angiotensin converting enzyme (ACE) inhibitors, certain calcium channel blockers, nitrates and endothelin-1 blocking agents. Calcium channel blockers and nitrates, while producing clinical improvement have not been clearly shown to prolong survival, whereas B-blockers and ACE inhibitors have been shown to significantly prolong life, as have aldosterone antagonists. Experimental studies using endothelin-1 blocking agents have shown a beneficial effect.
(ii) Systemic endothelial dysfunction is a well-recognized feature of HF and is clearly present by the time signs of left ventricular dysfunction are present. Endothelial dysfunction is important with respect to the intimate relationship of the myocardial microcirculation with cardiac myocytes. The evidence suggests that microvascular dysfunction contributes significantly to myocyte dysfunction and the morphological changes which lead to progressive myocardial failure.
   In terms of underlying pathophysiology, evidence suggests that endothelial dysfunction may be caused by a relative lack of NO which can be attributed to an increase in vascular O₂-formation by an NADH-dependent oxidase and subsequent excess scavenging of NO. Potential contributing factors to increased O₂-production include increased sympathetic tone, norepinephrine, angiotensin II, endothelin-1 and TNF-α. In addition, levels of IL-10, a key anti-inflammatory cytokine, are inappropriately low in relation to TNF-α levels. It is now believed that elevated levels of TNF-α, with associated proinflammatory cytokines including IL-6, and soluble TNF-α receptors, play a significant role in the evolution of HF by causing decreased myocardial contractility, biventricular dilatation, and hypotension and are probably involved in endothelial activation and dysfunction. It is also believed that TNF-α may play a role in the hitherto unexplained muscular wasting which occurs in severe HF patients. Preliminary studies in small numbers of patients with soluble TNF-receptor therapy have indicated improvements in NYHA functional classification and in patient well-being, as measured by quality of life indices.
(iii) Myocardial remodeling is a complex process which accompanies the transition from asymptomatic to symptomatic heart failure, and may be described as a series of adaptive changes within the myocardium, like alterations in ventricular shape, mass and volume (Piano, M.R., et al., J. Cardiovasc. Nurs. 14 (2000) 1-23; Molkentin, J.D., Ann. Rev. Physiol. 63 (2001) 391-426). The main components of myocardial remodeling are alterations in myocyte biology, like myocyte hypertrophy, loss of myocytes by necrosis or apoptosis, alterations in the extracellular matrix and alterations in left ventricular chamber geometry. It is unclear whether myocardial remodeling is simply the end-organ response that occurs following years of exposure to the toxic effects of long-term neurohormonal stimulation, or whether myocardial remodeling contributes independently to the progression of heart failure. Evidence to date suggests that appropriate therapy can slow or halt progression of myocardial remodeling.

### Markers and Disease State

As indicated above, myocyte hypertrophy is likely to represent one of the first steps down the road to HF. Myocyte hypertrophy is characterized by an increased expression of some genes encoding contractile proteins, such as p-myosin heavy chain and troponin T (TnT), and of some non-contractile proteins, such as A- type and B-type natriuretic peptides, by an increased cell size and by cytoskeletal alteration (Piano, M.R., et al., J. Cardiovasc. Nurs. 14 (2000) 1-23; Molkentin, J.D., Ann. Rev. Physiol. 63 (2001) 391-426).

European application EP1967853 A1 discloses a method for assessing heart failure (HF) in an individual comprising measuring in a sample obtained from the individual the concentration of the marker Nogo-C, wherein said sample is selected from the group consisting of serum, plasma, and whole blood.

Studies of human and animal models of heart failure suggest depressed mvocvte function in the later stages of cardiac failure. The mechanisms that underlie myocyte dysfunction have been suggested to involve alterations in the calcium-handling network, myofilament and cytoskeleton (de Tombe, P.P., Cardiovasc. Res. 37 (1998) 367-380). For example, in human and animal models of heart failure, sarcoplasmic reticulum calcium-ATPase enzyme activity is reduced, while both mRNA and protein levels of the sarcolemmal Na+/Ca2+ exchanger are increased. Moreover, there is isoform-switching of TnT, reduced phosphorylation of troponin I (TnI), decreased myofibrillar actomyosin ATPase activity and enhanced microtubule formation in both human and animal models of heart failure. Initially the changes to the heart, leading to myocardial remodeling are meant to compensate for the diseased parts of the myocardium in order to sustain the body's demand for oxygen and nutrients. However, the compensatory phase of heart failure is limited, and, ultimately, the failing heart is unable to maintain cardiac output adequate to meet the body's needs. Thus, there is a transition from a compensatory phase to a decompensatory phase. In the decompensatory phase, the cascade of changes in the heart continues but is no longer beneficial, moving the patient down the progression of heart failure to a chronic state and eventual death. According to the "ACC/AHA 2005 Guideline Update for the Diagnosis and Management of Chronic Heart Failure in the Adult" (S. Hunt et al., www.acc.org = the ACC/AHA practice guidelines) the disease continuum in the area of heart failure is nowadays grouped into four stages as noted above. In stages A and B the individuals at risk of developing heart failure are found, whereas stages C and D represent the groups of patients showing signs and symptoms of heart failure. Details for defining the different stages A through D as given in the above reference.

### Diagnostic Methods in Heart Failure

The single most useful diagnostic test in the evaluation of patients with HF is the comprehensive 2-dimensional echocardiogram coupled with Doppler flow studies to determine whether abnormalities of myocardium, heart valves, or pericardium are present and which chambers are involved. Three fundamental questions must be addressed: 1) is the LVEF preserved or reduced, 2) is the structure of the LV normal or abnormal, and 3) are there other structural abnormalities such as valvular, pericardial, or right ventricular abnormalities that could account for the clinical presentation? This information should be quantified with a numerical estimate of EF, measurement of ventricular dimensions and/or volumes, measurement of wall thickness, and evaluation of chamber geometry and regional wall motion. Right ventricular size and systolic performance should be assessed. Atrial size should also be determined semiquantitatively and left atrial dimensions and/or volumes measured.

Noninvasive hemodynamic data acquired at the time of echocardiography are an important additional correlate for patients with preserved or reduced EF. Combined quantification of the mitral valve inflow pattern, pulmonary venous inflow pattern, and mitral annular velocity provides data about characteristics of LV filling and left atrial pressure. Evaluation of the tricuspid valve regurgitant gradient coupled with measurement of inferior vena caval dimension and its response during respiration provides an estimate of systolic pulmonary artery pressure and central venous pressure.

Stroke volume may be determined with combined dimension measurement and pulsed Doppler in the LV outflow tract. However, abnormalities can be present in any of these parameters in the absence of HF. No one of these necessarily correlates specifically with HF; however, a totally normal filling pattern argues against clinical HF.

From a clinical perspective, the disease is clinically asymptomatic in the compensatory and early decompensatory phases (completely asymptomatic in stage A and with structural heart disease but no signs and symptoms of HF in stage B, cf. the ACC/AHA practice guidelines). Outward signs of the disease (such as shortness of breath) do not appear until well into the decompensatory phase (i.e., stages C and D according to the ACC/AHA guidelines). Current diagnosis is based on the outward symptoms of patients in stages C and D.

Typically patients with heart failure receive a standard treatment with drugs that interact with specific mechanisms involved in heart failure. There are no diagnostic tests that reflect those specific mechanisms reliably and help the physician to choose the right drug (and dose) for the right patient (e.g., ACE inhibitor, AT II, β-blockers, etc).

### Early assessment of patients for HF with Markers

Early assessment of patients at risk for heart failure appears to be possible only by biochemical markers since the individual at risk of developing heart failure at that stage is still free of clinical HF symptoms. There are no established biochemical markers currently available for the reliable pre-symptomatic assessment of the disease. By the time the diagnosis HF is established nowadays, the disease is already well under way.

The natriuretic peptide family, especially the atrial natriuretic peptide family and the brain natriuretic peptide family have in recent years proven to be of significant value in the assessment of HF.

### HF Prognosis and Need

At least partially due to the late diagnosis, 50% of patients with HF die within two years of diagnosis. The 5-year survival rate is less than 30%. There is a significant need for new biochemical markers aiding in the early diagnosis of heart failure.

An improvement in the early assessment of individuals at risk for heart failure, i.e., of individuals that are clinically asymptomatic for heart failure is warranted.

It has been established in recent years that B-type natriuretic peptide markers represent an excellent tool to monitor disease progression in patients with HF and to assess their risk of cardiovascular complications, like heart attack.

However, as for many other diagnostic areas a single marker is not sufficient.

Whereas a low value of NT-proBNP has a very high negative predictive value for ruling out HF or LVD, the positive predictive value for heart failure in the above and other studies (cf. Triepels R.H., et al., Clin. Chem. 49, Suppl. A (2003) 37-38) has been found to be in the range of 50-60%. Thus a marker useful in assessing individuals at risk for heart failure that on its own e.g., has a high, or in combination with NT-proBNP, and as compared to NT-proBNP alone has an improved positive predictive value for HF is of high clinical/practical importance.

A marker aiding in the assessment of a patient with heart failure also is of high importance to achieve further technical progress in this clinically very important and demanding diagnostic area.

### Summary of the Invention

It has now been found and established that the marker endostatin can aid in the diagnosis of heart failure.

Disclosed herein is a method for diagnosing heart failure in an individual comprising the steps of measuring in a sample obtained from the individual the concentration of the marker endostatin, of optionally measuring in the sample the concentration of one or more other marker(s) of heart failure, wherein said one or more other marker is selected from the group consisting of a natriuretic peptide marker, a cardiac troponin marker, and a marker of inflammation, and of assessing heart failure by comparing the concentration of endostatin and optionally the concentration(s) of the one or more other marker to the concentration of this marker or these markers as established in a control sample, wherein said sample is selected from the group consisting of serum, plasma, and whole blood.

The invention also relates to the use of protein endostatin as a marker molecule in the diagnosis of heart failure.

Further disclosed is the use of a marker combination comprising endostatin and one or more other marker of heart failure in the diagnosis of heart failure.

Also disclosed is a kit for performing the method for assessing heart failure in vitro comprising the steps of measuring in a sample the concentration of the marker endostatin, of optionally measuring in the sample the concentration of one or more other marker(s) of heart failure, and of assessing heart failure by comparing the concentration of endostatin and optionally the concentration(s) of the one or more other marker to the concentration of this marker or these markers as established in a reference population, the kit comprising the reagents required to specifically measure endostatin and the optionally one or more other marker of heart failure.

### Detailed Description of the Invention

In a first embodiment the present invention relates to a method for diagnosing heart failure in an individual comprising the steps of a) measuring in a sample obtained from the individual the concentration of the marker endostatin, b) optionally measuring in the sample the concentration of one or more other marker(s) of heart failure, and c) assessing heart failure by comparing the concentration determined in step (a) and optionally the concentration(s) determined in step (b) to the concentration of this marker or these markers as established in a control sample.

As used herein, each of the following terms has the meaning associated with it in this section.

The articles "a" and "an" are used herein to refer to one or to more than one (i.e., to at least one) of the grammatical object of the article. By way of example, "an antibody" means one antibody or more than one antibody.

The expression "one or more" denotes 1 to 50, preferably 1 to 20 also preferred 2, 3, 4, 5, 6, 7, 8, 9, 10, 12, or 15.

The term "marker" or "biochemical marker" as used herein refers to a molecule to be used as a target for analyzing a patient's test sample. In one embodiment examples of such molecular targets are proteins or polypeptides. Proteins or polypeptides used as a marker in the present invention are contemplated to include naturally occurring fragments of said protein in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. In addition, or in the alternative a marker polypeptide may carry a post-translational modification. Examples of posttranslational modifications amongst others are glycosylation, acylation, and/or phosphorylation.

The term "assessing heart failure" is used to indicate that the method will aid, e.g. a physician, to assess whether an individual is at risk of developing heart failure, or aid the physician in his assessing of an HF patient in one or several other areas of diagnostic relevance in HF. Preferred areas of diagnostic relevance in assessing an individual with HF are the staging of heart failure, differential diagnosis of acute and chronic heart failure, judging the risk of disease progression, guidance for selecting an appropriate drug, monitoring of response to therapy, and the follow-up of HF patients.

A "marker of heart failure" in the sense of the present invention is a marker that if combined with the marker endostatin adds relevant information in the assessment of HF to the diagnostic question under investigation. The information is considered relevant or of additive value if at a given specificity the sensitivity, or if at a given sensitivity the specificity, respectively, for the assessment of HF can be improved by including said marker into a marker combination already comprising the marker endostatin. Preferably the improvement in sensitivity or specificity, respectively, is statistically significant at a level of significance of p = 0.05, 0.02, 0.01 or lower. Preferably, the one or more other marker of heart failure is selected from the group consisting of a natriuretic peptide marker, a cardiac troponin marker, and a marker of inflammation.

The term "sample" as used herein refers to a biological sample obtained for the purpose of evaluation in vitro. In the methods of the present invention, the sample or patient sample preferably may comprise any body fluid. Preferred test samples include blood, serum, plasma, urine, saliva, and synovial fluid. Preferred samples are whole blood, serum, plasma or synovial fluid, with plasma or serum representing the most convenient type of sample. As the skilled artisan will appreciate, any such assessment is made in vitro. The patient sample is discarded afterwards. The patient sample is solely used for the in vitro method of the invention and the material of the patient sample is not transferred back into the patient's body. Typically, the sample is a liquid sample, e.g., whole blood, serum, or plasma.

The expression "comparing the concentration ... to the concentration as established in a control sample" is merely used to further illustrate what is obvious to the skilled artisan anyway. The control sample may be an internal or an external control sample. In one embodiment an internal control sample is used, i.e. the marker level(s) is(are) assessed in the test sample as well as in one or more other sample(s) taken from the same subject to determine if there are any changes in the level(s) of said marker(s). In another embodiment an external control sample is used. For an external control sample the presence or amount of a marker in a sample derived from the individual is compared to its presence or amount in an individual known to suffer from, or known to be at risk of, a given condition; or an individual known to be free of a given condition, i.e., "normal individual". For example, a marker level in a patient sample can be compared to a level known to be associated with a specific course of disease in HF. Usually the sample's marker level is directly or indirectly correlated with a diagnosis and the marker level is e.g. used to determine whether an individual is at risk for HF. Alternatively, the sample's marker level can e.g. be compared to a marker level known to be associated with a response to therapy in HF patients, the differential diagnosis of acute and chronic heart failure, the guidance for selecting an appropriate drug to treat HF, in judging the risk of disease progression, or in the follow-up of HF patients. Depending on the intended diagnostic use an appropriate control sample is chosen and a control or reference value for the marker established therein. It will be appreciated by the skilled artisan that such control sample in one embodiment is obtained from a reference population that is age-matched and free of confounding diseases. As also clear to the skilled artisan, the absolute marker values established in a control sample will be dependent on the assay used. Preferably samples from 100 well-characterized individuals from the appropriate reference population are used to establish a control (reference) value. Also preferred the reference population may be chosen to consist of 20, 30, 50, 200, 500 or 1000 individuals. Healthy individuals represent a preferred reference population for establishing a control value.

### Endostatin

Endostatin was originally isolated from murine hemangioendothelioma as a 20 kDA proteolytic fragment of type XVIII collagen (O'Reilly, M.S. et al., Cell 88 (1997) 277-285).

Collagens represent a family of extracellular matrix proteins with a characteristic triple-helical conformation forming supra-molecular aggregates that play a dominant role in maintaining tissue structural integrity. Excessive collagen deposition leads to fibrosis disrupting the normal functioning of surrounding tissues.

Collagen XVIII (COL18A1, FLJ27325, FLJ34914, KNO, KNO1, MGC74745, gxHOMSA10749, collagen, type XVIII, alpha 1) is a member of the Multiplexin family of collagens with multiple interruptions in the central triple-helical domain and a unique non-triple-helical domain at the C-terminus mainly in basement membranes. The sequence of the short isoform of human type alpha 1-chain of collagen XVIII (SwissProt: P39060) is given in SEQ ID NO:1.

Endostatin is released from the alpha 1 chain of collagen XVIII by action of various proteolytic enzymes (for details see Ortega, N. and Werb, Z., Journal of Cell Science 115 (2002) 4201-4214). Endostatin essentially is represented by the collagen XVIII fragment spanning from amino acid position 1337 to amino acid position 1519 of SEQ ID NO:1. The hinge region at the C-terminus of the alpha chain of collagen XVIII contains several protease sensitive sites and a number of enzymes, including neutrophil elastase, cathepsins and matrix metalloproteinases are known to generate endostatin by cleaving the collagen chain in this region. These proteases do not exclusively release endostatin but also may release other, larger fragments that contain the endostatin sequence. As obvious to the skilled artisan such larger fragments will also be measured by an immunoassay for endostatin

Physiological serum levels of monomeric endostatin (Antiangiogenic agent, multifunctional protein MFP) play a role in the stimulation of migration, proliferation, apoptosis or survival of different cell-types and in the suppression of various morphogenetic events (Eye morphogenesis and maturation of blood vessels).

Endostatin is a potent inhibitor of angiogenesis and blood vessel growth. The relationship between endostatin and cytokine networks is undetermined, but it is known that endostatin is able to alter expression of a wide range of genes (Abdollahi, A. et al., Mol. Cell 13 (2004) 649-663).

Disturbances of angiogenesis via imbalanced production of VEGF and endostatin result in the pathogenesis of angiogenesis-related diseases (Systemic sclerosis, atherothrombotic vascular disease, Preeclampsia).

Endostatin is known to inhibit endothelial proliferation and to suppress tumor growth. Elevated circulating levels of endostatin are associated with many forms of cancer (Dubois, S. et al., Cancer 109 (2007) 814-819).

In addition elevated endostatin levels were found in the alveolar space of patients with idiopathic pulmonary fibrosis (Richter, A.G. et al., Thorax 64 (2009) 156-161).

Little is known about the pathophysiology of endostatin in cardiac diseases. Preliminary data report changes of serum levels of endostatin in parallel with those of VEGF in response to myocardial ischaemia/ reperfusion (Seko, Y. et al., Clinical Science 106 (2004) 439-442). EP 1 719 779 relates to the therapeutic use of endostatin after myocardial infarction. Acute left ventricular heart failure has been described as a cardiac side-effect of treatment of a cancer patient with recombinant human endostatin (Jing, Q. et al., Clin.Oncol. 20 (2008) 268).

Differential expression of collagen XVIII mRNA was measured in patients suffering from endometriosis (US 2003/0077589).

Several patent applications deal with a potential utility of collagen XVIII or Endostatin polypeptides as diagnostic agents, preventive agents and therapeutic agents for a broad variety of diseases. For example, EP 1 925 940 claims that endostatin would be one of several serum markers predicting success of an anti-tumor treatment and WO 1998/056399 proposes the immunological detection of collagen XVIII in serum in association with liver fibrosis or hepatocellular carcinoma.

It would appear that in the prior art the presence or level of the protein endostatin in a bodily fluid is neither known to have nor suggested to have a diagnostic utility in the assessment of heart failure.

The inventors of the present invention have now found and could establish that an increased value for endostatin as measured from a bodily fluid sample derived from an individual is indicative for heart failure.

The values for endostatin as measured in a control group or a control population are for example used to establish a cut-off value or a reference range. A value above such cut-off value or out-side the reference range and its higher end is considered as elevated.

In a one embodiment a fixed cut-off value is established. Such cut-off value is chosen to match the diagnostic question of interest.

In one embodiment values for endostatin as measured in a control group or a control population are used to establish a reference range. In a preferred embodiment an endostatin concentration is considered as elevated if the value measured is above the 90%-percentile of the reference range. In further preferred embodiments an endostatin concentration is considered as elevated if the value measured is above the 95%-percentile, the 96%-percentile, the 97%-percentile or the 97.5%-percentile of the reference range.

In one embodiment the control sample will be an internal control sample. In this embodiment serial samples are obtained from the individual under investigation and the marker levels are compared. This may for example be useful in assessing the efficacy of therapy.

The method according to the present invention is based on a liquid sample which is obtained from an individual and on the measurement of endostatin in such sample. An "individual" as used herein refers to a single human or non-human organism. Thus, the methods and compositions described herein are applicable to both human and veterinary disease. Preferably the individual is a human being.

Preferably the marker endostatin is specifically measured from a liquid sample by use of a specific binding agent.

The liquid sample is selected from the group consisting of serum, plasma, and whole blood.

A specific binding agent is, e.g., a receptor for endostatin, a lectin binding to endostatin or an antibody to endostatin. A specific binding agent has at least an affinity of 10⁷ l/mol for its corresponding target molecule. The specific binding agent preferably has an affinity of 10⁸ l/mol or even more preferred of 10⁹ l/mol for its target molecule. As the skilled artisan will appreciate the term specific is used to indicate that other biomolecules present in the sample do not significantly bind to the binding agent specific for endostatin. Preferably, the level of binding to a biomolecule other than the target molecule results in a binding affinity which is only 10% or less, more preferably only 5% or less of the affinity to the target molecule, respectively. A preferred specific binding agent will fulfill both the above minimum criteria for affinity as well as for specificity.

A specific binding agent preferably is an antibody reactive with endostatin. The term antibody refers to a polyclonal antibody, a monoclonal antibody, antigen binding fragments of such antibodies, single chain antibodies as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays, Elsevier Science Publishers B.V., Amsterdam (1990), the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced (Tijssen, P., *supra,* pages 108-115).

For the achievements as disclosed in the present invention polyclonal antibodies raised in goats may be used. However, clearly also polyclonal antibodies from different species, e.g., rats, rabbits or guinea pigs, as well as monoclonal antibodies can be used. Since monoclonal antibodies can be produced in any amount required with constant properties, they represent ideal tools in development of an assay for clinical routine.

The generation and the use of monoclonal antibodies to endostatin in a method according to the present invention, respectively, represent yet other preferred embodiments.

It is not easy to purify endostatin from a natural source. The recombinant production of endostatin is a method of choice to obtain higher amounts of endostatin. In a preferred embodiment endostatin is produced by recombinant expression using an eukaryotic expression system. Examples of eukaryotic expression systems are baculovirus expression, expression in yeast and expression in a mammalian expression system. In one preferred embodiment the expression of endostatin will be performed in a mammalian expression system. Examples of mammalian expression systems are CHO cells, HEK cells, myeloma cells, etc. In a further preferred embodiment the recombinantly produced endostatin is used as an antigen in the production of poly- or monoclonal antibodies against endostatin. It may be also preferable to purify polyclonal antibodies by immunoadsorption over an endostatin immunoadsorber make use of a recombinantly produced endostatin as described herein above.

As the skilled artisan will appreciate now, that endostatin has been identified as a marker which is useful in the assessment of HF, alternative ways may be used to reach a result comparable to the achievements of the present invention. For example, alternative strategies to generate antibodies may be used. Such strategies comprise amongst others the use of synthetic or recombinant peptides, representing a clinically relevant epitope of endostatin for immunization. Alternatively, DNA immunization also known as DNA vaccination may be used.

For measurement the liquid sample obtained from an individual is incubated with the specific binding agent for endostatin under conditions appropriate for formation of a binding agent endostatin-complex. Such conditions need not be specified, since the skilled artisan without any inventive effort can easily identify such appropriate incubation conditions. The amount of binding agent endostatin-complex is measured and used in the assessment of HF. As the skilled artisan will appreciate there are numerous methods to measure the amount of the specific binding agent endostatin-complex all described in detail in relevant textbooks (cf., e.g., Tijssen P., *supra,* or Diamandis, E.P. and Christopoulos, T.K. (eds.), Immunoassay, Academic Press, Boston (1996)).

Preferably endostatin is detected in a sandwich type assay format. In such assay a first specific binding agent is used to capture endostatin on the one side and a second specific binding agent, which is labeled to be directly or indirectly detectable, is used on the other side. Preferably, an antibody to endostatin is used in a qualitative (endostatin present or absent) or quantitative (amount of endostatin is determined) immunoassay.

The inventors of the present invention surprisingly are able to detect protein endostatin in a bodily fluid sample. Even more surprising they are able to demonstrate that the presence of endostatin in such liquid sample obtained from an individual can be correlated to HF. No tissue and no biopsy sample is required to make use of the marker endostatin in the assessment of HF. Measuring the level of protein endostatin is considered very advantageous in the field of HF.

In a preferred embodiment the method according to the present invention is practiced with serum as liquid sample material. In a further preferred embodiment the method according to the present invention is practiced with plasma as liquid sample material. In a further preferred embodiment the method according to the present invention is practiced with whole blood as liquid sample material.

In a further preferred embodiment, the present invention relates to use of protein endostatin as a marker molecule in the assessment of heart failure from a liquid sample obtained from an individual.

The ideal scenario for diagnosis would be a situation wherein a single event or process would cause the respective disease as, e.g., in infectious diseases. In all other cases correct diagnosis can be very difficult, especially when the etiology of the disease is not fully understood as is the case of HF. As the skilled artisan will appreciate, no biochemical marker in the field of HF is diagnostic with 100% specificity and at the same time 100% sensitivity for a certain diagnostic question. Rather, biochemical markers are used to assess with a certain likelihood or predictive value an underlying diagnostic question. The skilled artisan is fully familiar with the mathematical/statistical methods that routinely are used to calculate a relative risk or likelihood for the diagnostic question to be assessed. In routine clinical practice various clinical symptoms and biological markers are generally considered together by a physician in the diagnosis, treatment, and management of the underlying disease.

Preferably in a further preferred embodiment of the present invention the method for assessment of HF is performed by measuring the concentration of endostatin and of one or more other marker and by using the concentration of endostatin and of the one or more other marker in the assessment of HF.

In the assessment of HF the marker endostatin will aid the physician in one or more of the following aspects: to assess an individual's risk for heart failure or to assess a patient having heart failure, e.g., with the intention to identify the stage of heart failure, to differentiate between acute and chronic heart failure, to judge the risk of disease progression, to provide guidance in selecting an appropriate therapy, to monitor a patient's response to therapy, and to monitor the course of disease, i.e., in the follow-up of HF patients.

### Screening (assessment whether individuals are at risk for developing heart failure)

The present disclosure relates to an in vitro method for assessing whether an individual is at risk for developing heart failure comprising the steps of measuring in a sample the concentration of the marker endostatin, of optionally measuring in the sample the concentration of one or more other marker(s) of heart failure, and of assessing said individual's risk for developing heart failure by comparing the concentration for endostatin and optionally the concentration(s) determined for the optionally one or more other marker(s) to the concentration of this marker or these markers to its or their reference value(s).

Screening relates to the unbiased assessment of individuals regarding their risk for developing heart failure. Whereas such screening may in theory be performed on any sample, in clinical practice such screening option will usually be given to individuals somehow at risk for development of heart failure. As discussed above, such individuals may clinically be asymptomatic, i.e., they have no signs or symptoms of HF. In one preferred embodiment screening for HF will be given to individuals at risk of developing heart failure, e.g. falling into the stages A or B as defined by the ACC/AHA practice guidelines.

As mentioned above, heart failure is one of the most prevalent, costly and life-threatening diseases in developed countries. Because of its high prevalence and its long asymptomatic phase identification of individuals at risk for developing HF would be of utmost importance to intervene in and if possible to interrupt the course of disease. Without a very early risk assessment, prevention of disease progression from the asymptomatic state into a symptomatic phase of HF appears impossible.

The risk for heart failure is assessed by mathematical/statistical methods fully known and understood by the skilled artisan. Preferably an individual's risk for heart failure is expressed in relative terms and given as the so-called relative risk (=RR). In order to calculate such RR for heart failure an individual's value for endostatin is compared to the values established for endostatin in a reference population, preferably healthy individuals not developing heart failure. Also preferred the assessment of such RR for heart failure is based on a group of individuals that have developed heart failure within the study period, preferably within one or also preferred within two years, and a group of individuals that did not develop heart failure in the same study period.

Disclosed is the use of the marker endostatin in the screening for heart failure. As the skilled artisan knows the term "use as a marker" implies that the concentration of a marker molecule is quantified by appropriate means and that value measured for such marker is then used to indicate, i.e. to mark, the presence or absence of a disease or clinical condition. Appropriate means for quantitation for example are specific binding agents, like antibodies.

Preferably the screening for HF will be performed in individuals suspected to be at risk of future heart failure. Patients at risk of future heart failure in this sense are patients diagnosed with hypertension, atherosclerotic disease, diabetes, obesity and metabolic syndrome. Preferably the risk for future heart failure is assessed with individuals suffering from hypertension, atherosclerotic disease, diabetes, and/or metabolic syndrome.

Also preferred is the use of the marker endostatin in assessing the risk for future heart failure for an individual in stage B according to the ACC/AHA practice guidelines, i.e., an individual exhibiting a structural change at the heart but not showing symptoms of heart failure.

Disclosed is the use of endostatin as one marker of a HF marker combination for HF screening purposes.

In the screening setting an elevated level of endostatin is a positive indicator for an individual's increased risk to develop heart failure.

### Staging of patients

The present disclosure relates to an in vitro method aiding in the staging of heart failure patients, comprising the steps of a) measuring in a sample the concentration of the marker endostatin, of b) optionally measuring in the sample the concentration of one or more other marker(s) of heart failure, and staging heart failure by comparing the concentration determined in step (a) and optionally the concentration(s) determined in step (b) to the concentration of this marker or these markers to its or their reference value(s). Preferably the level of marker endostatin is used as an aid in classifying the individuals investigated into the groups of individuals that are clinically "normal" (i.e., individuals in stage A according to the ACA/ACC classification), asymptomatic patients having structural heart disease (stage B according to the ACA/ACC classification) and the group of patients having heart failure (i.e., patients in stage C or stage D according to the ACA/ACC classification).

### Differentiation between an acute cardiac event and chronic cardiac disease

Disclosed is an in vitro method aiding in the differential diagnosis between an acute cardiac event and chronic cardiac disease, comprising the steps of measuring in a sample the concentration of the marker endostatin, of optionally measuring in the sample the concentration of one or more other marker(s) of heart failure, and establishing a differential diagnosis between an acute cardiac event and chronic cardiac disease by comparing the concentration determined in step (a) and optionally the concentration(s) determined in step (b) to the concentration of this marker or these markers to its or their reference value(s).

The person skilled in the art is familiar with the meanings of "acute cardiac event" and of "chronic cardiac disease".

Preferably, an "acute cardiac event" relates to an acute condition, disease or malfunction of the heart, particularly to acute heart failure, e.g., myocardial infarction (MI) or arrhythmia. Depending on the extent of an MI, it may be followed by LVD and CHF.

Preferably, a "chronic cardiac disease" is a weakening of heart function, e.g., due to ischemia of the heart, coronary artery disease, or previous, particularly small, myocardial infarction(s) (possibly followed by progressing LVD). It may also be a weakening due to inflammatory diseases, heart valve defects (e.g., mitral valve defects), dilatative cardiomyopathy, hypertrophic cardiomyopathy, heart rhythm defects (arrhythmias), and chronic obstructive pulmonary disease. Thus, it is clear that a chronic cardiac disease may also include patients who had suffered from an acute coronary syndrome, e.g., MI, but who are presently not suffering from an acute cardiac event.

It is important to differentiate between an acute cardiac event and chronic cardiac disease, because an acute cardiac event and chronic cardiac disease may require quite different treatment regimens. For example, for a patient presenting with acute myocardial infarction early treatment for reperfusion may be of utmost importance. Whereas a treatment for reperfusion performed on a patient with chronic heart failure at best is of no or only little harm to this patient.

The marker endostatin is used in the differential diagnosis of acute and chronic heart failure.

### Assessing the risk of disease progression

Disclosed is an in vitro method for assessing an HF-patient's risk for disease progression, comprising the steps of measuring in a sample the concentration of the marker endostatin, of optionally measuring in the sample the concentration of one or more other marker(s) of heart failure, and of establishing said individual's risk for disease progression by comparing the concentration for endostatin and optionally the concentration(s) determined for the optionally one or more other marker(s) to the concentration of this marker or these markers to its or their reference value(s).

At present it is very difficult to assess or to even predict with a reasonable likelihood whether a patient diagnosed with HF has a more or less stable status or whether the disease will progress and the patient's health status as result is likely to worsen. Severity and progression of heart failure is clinically usually established by assessing the clinical symptoms or by identification of adverse changes by using imaging technologies such as echocardiography. The worsening of heart failure is established by monitoring the left ventricular ejection fraction (LVEF). A deterioration in LVEF by 5% or more is considered as disease progression.

The present disclosure therefore relates to the use of the marker endostatin in assessing the risk of disease progression for a patient suffering from HF. In the assessment of disease progression for patients suffering from HF an elevated level of endostatin is an indicator for an increased risk of disease progression.

### Guidance in selecting an appropriate HF therapy

Disclosed is an in vitro method. aiding in the selection of an appropriate HF-therapy, comprising the steps of measuring in a sample the concentration of the marker endostatin, of optionally measuring in the sample the concentration of one or more other marker(s) of heart failure, and of selecting an appropriate therapy by comparing the concentration for endostatin and optionally the concentration(s) determined for the optionally one or more other marker(s) to the concentration of this marker or these markers to its or their reference value(s).

It is expected that the marker endostatin will be of help in aiding the physician to select the most appropriate treatment regimen from the various treatment regimens at hand in the area of heart failure. The present disclosure therefore relates to the use of the marker endostatin in selecting a treatment regimen for a patient suffering from HF.

### Monitor a patient's response to therapy

The present disclosure relates to an in vitro method for monitoring a patient's response to HF-therapy, comprising the steps of a) measuring in a sample the concentration of the marker endostatin, of b) optionally measuring in the sample the concentration of one or more other marker(s) of heart failure, and of monitoring a patient's response to HF-therapy by comparing the concentration determined in step (a) and optionally the concentration(s) determined in step (b) to the concentration of this marker or these markers to its or their reference value(s).

Alternatively the above method for motoring a patient's response to therapy can be practiced by establishing the pre- and post-therapeutic marker level for endostatin and for the optionally one or more other marker and by comparing the pre- and the post-therapeutic marker level(s).

The diagnosis of heart failure is clinically established. According to the present invention HF is considered clinically established if a patient meets the criteria of stages C or D as defined by the ACC/AHA practice guidelines. According to these guidelines stage C refers to patients with structural heart disease and with prior or current symptoms of heart failure. Patients in stage D are those patients with refractory heart failure that require specialized interventions.

As indicated further above the values measured for NT-proBNP are highly correlated to the severity of heart failure. However, both BNP and NT-proBNP appear to be not ideal in monitoring a patient's response to therapy, cf. e.g., Beck-da-Silva, L., et al., Congest. Heart Fail. 11 (2005) 248-253, quiz 254-255.

The marker endostatin appears to be appropriate to monitor a patient's response to therapy. In that diagnostic area the marker endostatin can also be used for establishing a baseline value before therapy and to measure endostatin at one time-point or several time-points after therapy. In the follow-up of HF patients a reduced level of endostatin is a positive indicator for an effective treatment of HF.

### Marker combination

Biochemical markers can either be determined individually or, in a preferred embodiment of the invention, they can be measured simultaneously using a chip- or a bead-based array technology. The concentrations of the biomarkers are then interpreted independently using an individual cut-off for each marker or they are combined for interpretation, i.e., they form a marker combination.

As the skilled artisan will appreciate the step of correlating a marker level to a certain likelihood or risk can be performed and achieved in different ways. Preferably the values measured for the marker endostatin and the one or more other marker(s) are mathematically combined and the combined value is correlated to the underlying diagnostic question. Marker values may be combined with the measurement of endostatin by any appropriate state of the art mathematical method.

Preferably the mathematical algorithm applied in the combination of markers is a logistic function. The result of applying such mathematical algorithm or such logistical function preferably is a single value. Dependent on the underlying diagnostic question such value can easily be correlated to e.g., the risk of an individual for heart failure or to other intended diagnostic uses helpful in the assessment of patients with HF. In a preferred way such logistic function is obtained by a) classification of individuals into the groups, e.g., into normals, individuals at risk for heart failure, patients with acute or chronic heart failure and so on, b) identification of markers which differ significantly between these groups by univariate analysis, c) logistic regression analysis to assess the independent discriminative values of markers useful in assessing these different groups and d) construction of the logistic function to combine the independent discriminative values. In this type of analysis the markers are no longer independent but represent a marker combination.

In a preferred embodiment the logistic function used for combining the values for endostatin and the value of at least one further marker is obtained by a) classification of individuals into the groups of normals and individuals at risk of heart failure, respectively, b) establishing the values for endostatin and the value of the at least one further marker c) performing logistic regression analysis and d) construction of the logistic function to combine the marker values for endostatin and the value of the at least one further marker.

A logistic function for correlating a marker combination to a disease preferably employs an algorithm developed and obtained by applying statistical methods. Appropriate statistical methods e.g. are Discriminant analysis (DA) (i.e., linear-, quadratic-, regularized-DA), Kernel Methods (i.e., SVM), Nonparametric Methods (i.e., k-Nearest-Neighbor Classifiers), PLS (Partial Least Squares), Tree-Based Methods (i.e., Logic Regression, CART, Random Forest Methods, Boosting/Bagging Methods), Generalized Linear Models (i.e., Logistic Regression), Principal Components based Methods (i.e., SIMCA), Generalized Additive Models, Fuzzy Logic based Methods, Neural Networks and Genetic Algorithms based Methods. The skilled artisan will have no problem in selecting an appropriate statistical method to evaluate a marker combination of the present invention and thereby to obtain an appropriate mathematical algorithm. Preferably the statistical method employed to obtain the mathematical algorithm used in the assessment of heart failure is selected from DA (i.e., Linear-, Quadratic-, Regularized Discriminant Analysis), Kernel Methods (i.e., SVM), Nonparametric Methods (i.e., k-Nearest-Neighbor Classifiers), PLS (Partial Least Squares), Tree-Based Methods (i.e., Logic Regression, CART, Random Forest Methods, Boosting Methods), or Generalized Linear Models (i.e., Logistic Regression). Details relating to these statistical methods are found in the following references: Ruczinski, I., et al., J. of Computational and Graphical Statistics 12 (2003) 475-511; Friedman, J.H., J. of the American Statistical Association 84 (1989) 165-175; Hastie, T., et al., The Elements of Statistical Learning, Springer Verlag (2001); Breiman, L., et al., Classification and regression trees, Wadsworth International Group, California (1984); Breiman, L., Machine Learning 45 (2001) 5-32; Pepe, M.S., The Statistical Evaluation of Medical Tests for Classification and Prediction, Oxford Statistical Science Series, 28, Oxford University Press (2003); and Duda, R.O., et al., Pattern Classification, John Wiley & Sons, Inc., 2nd ed. (2001).

It is a preferred embodiment of the invention to use an optimized multivariate cut-off for the underlying combination of biological markers and to discriminate state A from state B, e.g., normals and individuals at risk for heart failure, HF patients responsive to therapy and therapy failures, patients having an acute heart failure and HF patients having chronic heart failure, HF patients showing disease progression and HF patients not showing disease progression, respectively.

The area under the receiver operator curve (=AUC) is an indicator of the performance or accuracy of a diagnostic procedure. Accuracy of a diagnostic method is best described by its receiver-operating characteristics (ROC) (see especially Zweig, M.H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example, health and disease or disease progression versus no disease progression.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot (AUC). By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

The overall assay sensitivity will depend on the specificity required for practicing the method disclosed here. In certain preferred settings a specificity of 75% may be sufficient and statistical methods and resulting algorithms can be based on this specificity requirement. In one preferred embodiment the method used to assess individuals at risk for heart failure is based on a specificity of 80%, of 85%, or also preferred of 90% or of 95%.

As discussed above, the marker endostatin aids in assessing an individuals risk of developing heart failure as well as in the further in vitro diagnostic assessment of a patient having heart failure. A preferred embodiment accordingly is the use of endostatin as a marker molecule in the assessment of heart failure.

The use of a marker combination comprising endostatin and one or more other marker(s) of HF in the assessment of HF patients or in the assessment of individuals at risk for HF represents a further preferred embodiment of the present invention. In such marker combination the one or more other marker(s) preferably is selected from the group consisting of a natriuretic peptide marker, a cardiac troponin marker, and a marker of inflammation.

The one or more preferred selected other HF marker(s) with which the measurement of endostatin may be combined preferably is or are selected from the group consisting of a natriuretic peptide marker, a cardiac troponin marker, and a marker of inflammation. These preferred other markers whose measurement(s) preferably are combined with the measurement of endostatin or which form part of the HF marker combination comprising endostatin, respectively, are discussed in more detail below.

### Natriuretic peptide marker

A natriuretic peptide marker in the sense of the present invention is either a marker selected from the atrial natriuretic peptide (ANP) family or a marker selected from the brain natriuretic peptide (BNP) family.

The polypeptide markers in either the atrial natriuretic peptide family or in the brain natriuretic peptide family are derived from the preproforms of the corresponding active hormones.

Preferred natriuretic peptide markers according to the present invention are NT-proANP, ANP, NT-proBNP, BNP, and immunologically detectable physiological fragments thereof. As the skilled artisan readily appreciates, the immunologically detectable fragment has to comprise at least one epitope allowing for the specific detection of such physiological fragment. A physiological fragment is a fragment as naturally present in an individual's circulation.

The markers in both the natriuretic peptide families represent fragments of the corresponding pro-hormones, i.e., proANP and proBNP, respectively. Since similar considerations apply for both families, only the BNP marker family shall be described in some detail. The pro-hormone of the BNP family, i.e., proBNP consists of 108 amino acids. proBNP is cleaved into the 32 C-terminal amino acids (77-108) representing the biologically active hormone BNP and the N-terminal amino acids 1-76 called N-terminal proBNP (or NT-proBNP). BNP, N-terminal proBNP (1-76) as well as further breakdown products (Hunt, P.J., et al., Biochem. Biophys. Res. Com. 214 (1995) 1175-1183) circulate in blood. Whether the complete precursor molecule (proBNP 1-108) also occurs in the plasma is not completely resolved. It is however described (Hunt, P.J., et al., Peptides 18 (1997) 1475-1481) that a low release of proBNP (1-108) in plasma is detectable but that due to the very quick partial breakdown at the N-terminal end some amino acids are absent. Today it is generally accepted that e.g., for NT-proBNP the central portion of the molecule, residing in between the amino acids 10 to 50 represents a physiologically rather stable part. NT-proBNP molecules comprising this central part of NT-proBNP can be reliably measured from bodily fluids. Detailed disclosure relating to methods based on the immunological detection of this central part of the NT-proBNP molecule is given in WO 00/45176 and the reader is referred thereto for details. It may be of further advantage to measure only a certain subfraction of NT-proBNP for which the term native NT-proBNP has been proposed. Detailed disclosure relating to this subtraction of NT-proBNP is found in WO 2004/099253. The artisan will find all necessary instructions there. Preferably the NT-proBNP measured is or corresponds to the NT-proBNP as measured with the Elecsys® NT-proBNP assay from Roche Diagnostics, Germany.

Preanalytics are robust with NT-proBNP, which allows easy transportation of the sample to a central laboratory (Mueller, T., et al., Clin. Chem. Lab. Med. 42 (2004) 942-944). Blood samples can be stored at room temperature for several days or may be mailed or shipped without recovery loss. In contrast, storage of BNP for 48 hours at room temperature or at 4° Celsius leads to a concentration loss of at least 20 % (Mueller, T., et al., supra; Wu, A.H., et al., Clin. Chem. 50 (2004) 867-873).

The brain-derived natriuretic peptide family (especially BNP and NT-proBNP) has been thoroughly investigated in the screening of certain populations for HF. The findings with these markers, especially with NT-proBNP are quite encouraging. Elevated values of NT-proBNP even in asymptomatic "patients" are clearly indicative for "heart problems" (Gremmler, B., et al., Exp. Clin. Cardiol. 8 (2003) 91-94). These authors showed that an elevated NT-proBNP indicates the presence of 'cardio-renal distress' and should prompt referral for further investigation. In line with several other groups of investigators Gremmler, et al., also find that an abnormal NT-proBNP concentration is an accurate diagnostic test both for the exclusion of HF in the population and in ruling out left ventricular dysfunction (=LVD) in breathless subjects. The role of negative BNP or NT-proBNP values in ruling out HF or LVD is corroborated by other groups of investigators, cf., e.g., McDonagh, T.A., et al., Eur. J. Heart Fail. 6 (2004) 269-273; and Gustafsson, F., et al., J. Card. Fail. 11, Suppl. 5 (2005) S15-20.

BNP is produced predominantly (albeit not exclusively) in the ventricle and is released upon increase of wall tension. Thus, an increase of released BNP reflects predominantly dysfunctions of the ventricle or dysfunctions which originate in the atria but affect the ventricle, e.g., through impaired inflow or blood volume overload. In contrast to BNP, ANP is produced and released predominantly from the atrium. The level of ANP may therefore predominantly reflect atrial function.

ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NT-proBNP is 120 min longer than that of BNP, which is 20 min (Smith, M.W., et al., J. Endocrinol. 167 (2000) 239-246).

Therefore, depending on the time-course or properties of interest, either measurement of the active or the inactive forms of the natriuretic peptide can be advantageous.

In the assessment of an individual at risk for heart failure the value measured for endostatin is preferably combined with the value for NT-proANP and/or NT-proBNP. Preferably the value for NT-proBNP is combined with the value for endostatin. Similar considerations apply for selecting an appropriate therapy, judging the risk of disease progression, and to monitoring the course of disease.

In case endostatin is used in assessing a patient's response to therapy its measurement is preferably combined with the measurement of ANP or BNP.

In case endostatin is used to differentiate between acute and chronic heart failure the preferred marker combination comprises endostatin, ANP or proANP and BNP or proBNP.

### Cardiac troponin marker

The term cardiac troponin relates to the cardiac isoforms of troponin I and troponin T. As already indicated above the term marker also relates to physiological variants of the marker molecule, like physiological fragments or complexes. For the cardiac troponin markers their physiologically occurring complexes are known to be of diagnostic relevance and are herewith expressly included.

Troponin T has a molecular weight of about 37.000 Da. The troponin T isoform that is found in cardiac tissue (cTnT) is sufficiently divergent from skeletal muscle TnT to allow for the production of antibodies that distinguish both these TnT isoforms. TnT is considered a marker of acute myocardial damage; cf. Katus, H.A., et al., J. Mol. Cell. Cardiol. 21 (1989) 1349-1353; Hamm, C.W., et al., N. Engl. J. Med. 327 (1992) 146-150; Ohman, E.M., et al., N. Engl. J. Med. 335 (1996) 1333-1341; Christenson, R.H., et al., Clin. Chem. 44 (1998) 494-501; and EP 0 394 819.

Troponin I (TnI) is a 25 kDa inhibitory element of the troponin complex, found in muscle tissue. TnI binds to actin in the absence of Ca²⁺, inhibiting the ATPase activity of actomyosin. The TnI isoform that is found in cardiac tissue (cTnI) is 40% divergent from skeletal muscle TnI, allowing both isoforms to be immunologically distinguished. The normal plasma concentration of cTnI is <0.1 ng/ml (4 pM). cTnI is released into the bloodstream following cardiac cell death; thus, the plasma cTnI concentration is elevated in patients with acute myocardial infarction (Benamer, H., et al., Am. J. Cardiol. 82 (1998) 845-850).

The unique cardiac isoforms of troponin I and T allow them to be distinguished immunologically from the other troponins of skeletal muscle. Therefore, the release into the blood of troponin I and T from damaged heart muscle can be specifically related to damage of cardiac tissue. It is nowadays also appreciated by the skilled artisan that the cardiac troponins may be detected from the circulation either in their free form or as a part of a complex (cf. e.g., US 6,333,397, US 6,376,206 and US 6,174, 686).

In the assessment of an individual at risk for heart failure as well as in the assessment of a patient suffering from heart failure, the value measured for endostatin is preferably combined with the value for cardiac isoform of troponin T and/or troponin I. A preferred cardiac troponin used in combination with the marker endostatin is cardiac troponin T.

### Marker of inflammation

The skilled artisan is familiar with the term marker of inflammation. Preferred markers of inflammation are interleukin-6, C-reactive protein, serum amyloid A and a S100 protein.

Interleukin-6 (IL-6) is a 21 kDa secreted protein that has numerous biological activities that can be divided into those involved in hematopoiesis and into those involved in the activation of the innate immune response. IL-6 is an acute-phase reactant and stimulates the synthesis of a variety of proteins, including adhesion molecules. Its major function is to mediate the acute phase production of hepatic proteins, and its synthesis is induced by the cytokines IL-1 and TNF-□. IL-6 is normally produced by macrophages and T lymphocytes. The normal serum concentration of IL-6 is < 5 pg/ml.

C-reactive protein (CRP) is a homopentameric Ca²⁺-binding acute phase protein with 21 kDa subunits that is involved in host defense. CRP synthesis is induced by IL-6, and indirectly by IL-1, since IL-1 can trigger the synthesis of IL-6 by Kupffer cells in the hepatic sinusoids. The normal plasma concentration of CRP is < 3µg/ml (30 nM) in 90% of the healthy population, and < 10 µg/ml (100 nM) in 99% of healthy individuals. Plasma CRP concentrations can, e.g., be measured by Serum amyloid A (=SAA) is an acute phase protein of low molecular weight of 11.7 kDa. It is predominantly synthesized by the liver in response to IL-1, IL-6 or TNF-α stimulation and is involved in the regulation of the T-cell dependent immune response. Upon acute events the concentration of SAA increases up to 1000-fold reaching one milligram per milliliter. It is used to monitor inflammation in diseases as divers as cystic fibrosis, renal graft refection, trauma or infections. In rheumatoid arthritis is has in certain cases been used as a substitute for CRP, but, SAA is not yet as widely accepted.

S100-proteins form a constantly increasing family of Ca²⁺-binding proteins that today includes more than 20 members. The physiologically relevant structure of S100-proteins is a homodimer but some can also form heterodimers with each other, e.g., S100A8 and S100A9. The intracellular functions range from regulation of protein phosphorylation, of enzyme activities, or of the dynamics of the cytoskeleton to involvement in cell proliferation and differentiation. As some S100-proteins are also released from cells, extracellular functions have been described as well, e.g., neuronal survival, astrocyte proliferation, induction of apoptosis and regulation of inflammatory processes. S100A8, S100A9, the heterodimer S100A8/A9 and S100A12 have been found in inflammation with S100A8 responding to chronic inflammation, while S100A9, S100A8/A9 and S100A12 are increased in acute inflammation. S100A8, S100A9, S100A8/A9 and S100A12 have been linked to different diseases with inflammatory components including some cancers, renal allocraft rejection, colitis and most importantly to RA (Burmeister, G., and Gallacchi, G., Inflammopharmacology 3 (1995) 221-230; Foell, D., et al., Rheumathology 42 (2003) 1383-1389). The most preferred S100 markers for assessing an individual at risk for HF or a patient having HF e.g., for use in a marker combination according to the present invention are S100A8, S100A9, S100A8/A9 heterodimer and S100A12.

sE-selectin (soluble endothelial leukocyte adhesion molecule-1, ELAM-1) is a 115 kDa, type-I transmembrane glycoprotein expressed only on endothelial cells and only after activation by inflammatory cytokines (IL-1β, TNF-α) or endotoxin. Cell-surface E-selectin is a mediator of the rolling attachment of leucocytes to the endothelium, an essential step in extravasion of leucocytes at the site of inflammation, thereby playing an important role in localized inflammatory response. Soluble E-selectin is found in the blood of healthy individuals, probably arising from proteolytic cleavage of the surface-expressed molecule. Elevated levels of sE-selectin in serum have been reported in a variety of pathological conditions (Gearing, A.J. and Hemingway, I., Ann. N.Y. Acad. Sci. 667 (1992) 324-331).

In a preferred embodiment the present invention relates to the use of endostatin as a marker molecule for HF in combination with one or more marker molecule(s) for HF, wherein said one or more other marker is selected from the group consisting of a natriuretic peptide marker, a cardiac troponin marker, and a marker of inflammation, in the diagnosis of HF from a liquid sample obtained from an individual, wherein said sample is selected from the group consisting of serum, plasma, and whole blood.

As indicated above, in a preferred method according to the present invention the value measured for endostatin is at least combined with the value of at least one further marker selected from the group consisting of a natriuretic peptide marker, a cardiac troponin marker, and a marker of inflammation.

In a preferred embodiment the present invention relates to the use of the marker combination endostatin and NT-proBNP in the diagnosis of heart failure.

In a preferred embodiment the present invention relates to the use of the marker combination endostatin and troponin T in the diagnosis of heart failure.

Also disclosed is the use of the marker combination endostatin and CRP in the assessment of heart failure.

Also disclosed is a marker combination comprising the markers endostatin, troponin T, NT-proBNP and CRP. In yet a further preferred embodiment the present invention relates to a marker panel used in a method for diagnosing HF in vitro by biochemical markers, comprising measuring in a sample the concentration of endostatin and of one or more other marker of HF, wherein said one or more other marker is selected from the group consisting of a natriuretic peptide marker, a cardiac troponin marker, and a marker of inflammation, and using the concentrations determined in the diagnosis of HF.

A marker panel according to the present invention is preferably measured using a protein array technique. An array is a collection of addressable individual markers. Such markers can be spacially addressable, such as arrays contained within microtiter plates or printed on planar surfaces where each marker is present at distinct X and Y coordinates. Alternatively, markers can be addressable based on tags, beads, nanoparticles, or physical properties. The microarrays can be prepared according to the methods known to the ordinarily skilled artisan (see for example, US 5,807,522; Robinson, W.H., et al., Nat. Med. 8 (2002) 295-301; Robinson, W.H., et al., Arthritis Rheum. 46 (2002) 885-893). Array as used herein refers to any immunological assay with multiple addressable markers. In one embodiment the addressable markers are antigens. In another embodiment the addressable elements are autoantibodies. A microarray is a miniaturized form of an array. Antigen as used herein refers to any molecule that can bind specifically to an antibody. The term autoantibody is well-defined in the art.

A protein array comprising the marker endostatin and optionally one or more other marker of HF, wherein said one or more other marker is selected from the group consisting of a natriuretic peptide marker, a cardiac troponin marker, and a marker of inflammation, is disclosed.

Also disclosed is a protein array comprising the markers endostatin and NT-proBNP.

Also disclosed are a protein array comprising the markers endostatin and troponin T, a protein array comprising the markers endostatin and CRP, and a protein array comprising the markers endostatin, troponin T, NT-proBNP and CRP.

Also disclosed is a kit comprising the reagents required to specifically measure endostatin. Also preferred is a kit comprising the reagents required to specifically measure endostatin and the reagents required to measure the one or more other marker of heart failure that are used together in an HF marker combination.

The following examples, sequence listing and figures are provided to aid the understanding of the present invention, the true scope of which is set forth in the appended claims.

### Description of the Sequence Listing

**SEQ ID NO:1** Amino acid sequence of the short isoform of the alpha 1 chain of human collagen type XVIII.

### Description of the Figures

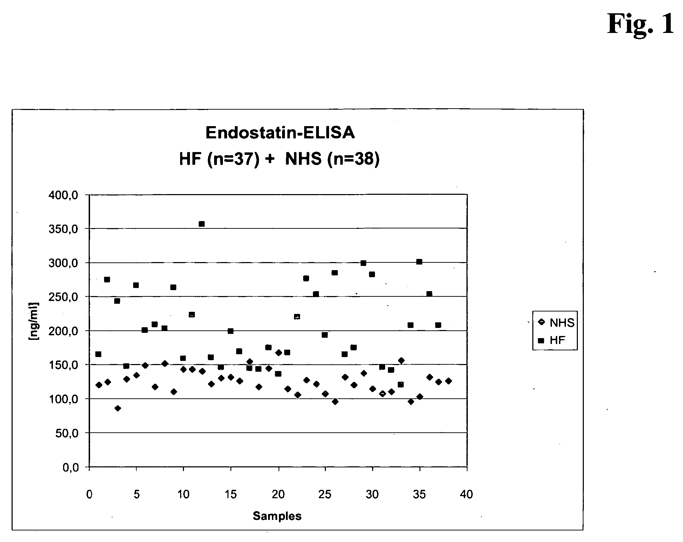

- **Figure 1**: **Endostatin as measured in samples from 37 HF patients and 38 control samples, respectively.** Concentrations in ng/ml are given on the y-axis. Samples derived from patients with heart failure are labeled HF (HF = squares), and NHS for healthy controls (normal human serum = NHS = rhombi), respectively.

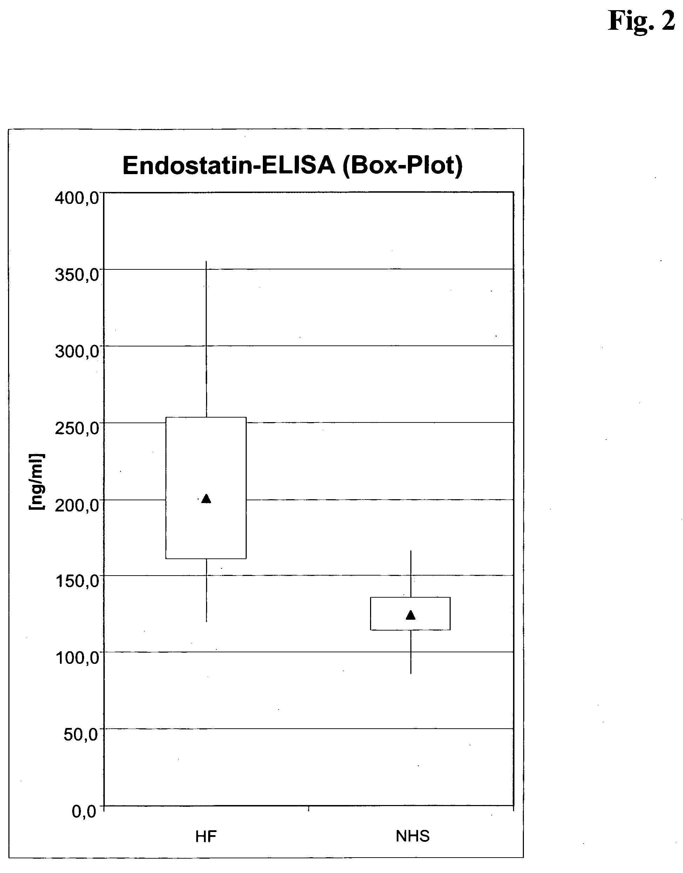

- **Figure 2**: **Endostatin was measured in samples from 37 HF patients and 38 control samples, respectively.** Quartiles have been calculated based on concentrations in ng/ml both for the samples derived from patients with heart failure, labeled HF (HF = squares), and the samples from healthy controls (normal human serum = NHS = rhombi), respectively. The box-and-whisker-blots show the lower and upper quartiles (boxes) as well as the highest and lowest values (whiskers).

### Example 1

### 1.1 ELISA for the measurement of endostatin in human serum and plasma samples

For measurement of endostatin in human serum or plasma, a commercially available sandwich ELISA (Quantikine Human Endostatin Immunoassay, Catalog Number DNST0, R&D Systems) is used. Measurements are performed according to the instructions given by the manufacturer.

### 1.2 Endostatin ELISA with sera of patients have HF and obtained out of the clinical routine and apparently healthy donors, respectively

In order to further evaluate the utility of the endostatin assays under routine clinical conditions a panel of sera from HF patients (n=37 - see Table 1) and of 38 sera from apparently healthy control patients (see Table 2) is investigated.

Table 1 shows the result for patients with heart failure.

**Table 1:**

| **Endostatin ELISA results (panel with HF samples from clinical routine)** | |
|---|---|
| **Heart Failure Samples** | |
| **Sample No.** | **Endostatin-ELISA [ng/ml]** |
| 5078 | 163,8 |
| 5084 | 274,1 |
| 5085 | 243,6 |
| 5100 | 147,6 |
| 5101 | 265,8 |
| 5104 | 200,1 |
| 5107 | 208,0 |
| 5112 | 203,4 |
| 5113 | 262,3 |
| 5114 | 158,3 |
| 5301 | 223,5 |
| 5311 | 355,7 |
| 5314 | 160,2 |
| 5328 | 146,2 |
| 5382 | 198,7 |
| 5388 | 169,1 |
| 5397 | 144,5 |
| 5410 | 142,4 |
| 5421 | 174,1 |
| 5423 | 135,7 |
| 5439 | 167,8 |
| 5444 | 220,2 |
| 5449 | 275,8 |
| 5451 | 253,5 |
| 5455 | 192,7 |
| 5464 | 284,0 |
| 5469 | 164,1 |
| 5472 | 173,8 |
| 5474 | 298,4 |
| 5481 | 281,0 |
| 5482 | 145,2 |
| 5483 | 141,6 |
| 5491 | 119,9 |
| 5495 | 206,6 |
| 5502 | 300,3 |
| 5516 | 252,5 |
| 5517 | 207,6 |
| **Mean** | 207,1 |

Table 2 shows the endostatin results for healthy volunteers.

**Table 2:**

| **Endostatin ELISA results (samples from healthy controls)** | | |
|---|---|---|
| **NHS (Healthy Controls)** | | |
| | **No.** | **Endostatin-ELISA [ng/ml]** |
| **NHS panel 1** | 9 | 120,6 |
| | 16 | 123,7 |
| | 19 | 85,5 |
| | 29 | 128,5 |
| | 30 | 133,6 |
| | 32 | 149,0 |
| | 39 | 116,8 |
| | 40 | 151,7 |
| | 44 | 109,9 |
| | 45 | 142,8 |
| | 46 | 142,2 |
| | 47 | 139,5 |
| | 53 | 121,8 |
| | 68 | 129,8 |
| | 72 | 131,1 |
| | 73 | 125,3 |
| | 74 | 153,6 |
| | 78 | 117,3 |
| | 79 | 144,5 |
| | 80 | 166,5 |
| **NHS panel 2** | 1 | 114,5 |
| | 2 | 106,0 |
| | 3 | 127,3 |
| | 4 | 120,8 |
| | 5 | 106,5 |
| | 6 | 95,4 |
| | 7 | 132,1 |
| | 8 | 120,6 |
| | 9 | 137,0 |
| | 10 | 114,0 |
| | 11 | 107,8 |
| | 13 | 109,8 |
| | 14 | 156,2 |
| | 16 | 96,0 |
| | 17 | 102,8 |
| | 18 | 131,9 |
| | 19 | 123,8 |
| | 20 | 125,1 |
| | **Mean** | 125,3 |

The data summarized in Tables 1 and 2, respectively, are also shown graphically in Figure 1. The data of these Tables have also been used to calculate the box-blots shown in Figure 2. Figures 1 and 2 demonstrate that there is quite a difference in the average endostatin values as measured in sera derived from patients with heart failure as compared to endostatin values as measured in sera derived from apparently healthy control individuals.

### Example 2

### Marker combinations comprising the marker endostatin in the assessment of heart failure

### Example 2.1

### The marker combination NT-proBNP and endostatin

The marker combination NT-proBNP and endostatin is evaluated for the differentiation of patients in stage B and stages C plus D, respectively. Diagnostic accuracy is assessed by analyzing individual liquid samples obtained from well-characterized groups of individuals, i.e., 50 individuals in stage B according to the ACA/ACC criteria for classification of HF and 50 patients suffering from HF and having stage C according to the ACA/ACC criteria for classification of HF. NT-proBNP as measured by a commercially available assay (Roche Diagnostics, NT-proBNP-assay (Cat. No. 03 121 640 160 for Elecsys^{®} Systems immunoassay analyzer) and endostatin measured as described above are quantified in a serum sample obtained from each of these individuals. ROC-analysis is performed according to Zweig, M.H., and Campbell, G., *supra.* Discriminatory power for differentiating patients in stage C from individuals in stage B for the combination of endostatin with the established marker NT-proBNP is calculated by regularized discriminant analysis (Friedman, J. H., Regularized Discriminant Analysis, Journal of the American Statistical Association 84 (1989) 165-175).

### Example 2.2

### The marker combination troponin T and endostatin

The marker combination troponin T and endostatin is evaluated for the differentiation of patients suffering from an acute cardiac event from patients suffering from chronic heart disease, respectively. Diagnostic accuracy is assessed by analyzing individual liquid samples obtained from well-characterized groups of individuals, i.e., 50 individuals diagnosed as having an acute cardiac event and 50 individuals diagnosed as having chronic cardiac disease. Troponin T as measured by a commercially available assay (Roche Diagnostics, troponin T-assay (Cat. No. 201 76 44 for Elecsys^{®} Systems immunoassay analyzer) and endostatin measured as described above are quantified in a serum sample obtained from each of these individuals. ROC-analysis is performed according to Zweig, M. H., and Campbell, G., *supra.* Discriminatory power for differentiating patients in stage C from individuals in stage B for the combination of endostatin with the established marker troponin T is calculated by regularized discriminant analysis (Friedman, J.H., J. of the American Statistical Association 84 (1989) 165-175).

### Example 2.3

### The marker combination endostatin and CRP

The marker combination C-reactive protein and endostatin is evaluated for the differentiation of patients diagnosed as having a cardiomyopathy versus controls not suffering from any confounding heart disease, respectively. Diagnostic accuracy is assessed by analyzing individual liquid samples obtained from well-characterized groups of 50 individuals with cardiomyopathy and of 50 healthy control individuals. CRP as measured by a commercially available assay (Roche Diagnostics, CRP-assay (Tina-quant C-reactive protein (latex) high sensitive assay - Roche Cat. No. 11972855 216) and endostatin measured as described above are quantified in a serum sample obtained from each of these individuals. ROC-analysis is performed according to Zweig, M.H., and Campbell, G., *supra.* Discriminatory power for differentiating patients in stage C from individuals in stage B for the combination of endostatin with the established marker CRP is calculated by regularized discriminant analysis (Friedman, J.H., J. of the American Statistical Association 84 (1989) 165-175).

## Claims

1. A method for diagnosing heart failure in an individual comprising the steps of
a) measuring in a sample obtained from the individual the concentration of the marker endostatin,
b) optionally measuring in the sample the concentration of one or more other marker(s) of heart failure, wherein said one or more other marker is selected from the group consisting of a natriuretic peptide marker, a cardiac troponin marker, and a marker of inflammation and
c) diagnosing heart failure by comparing the concentration determined in step (a) and optionally the concentration(s) determined in step (b) to the concentration of this marker or these markers as established in a control sample,
wherein said sample is selected from the group consisting of serum, plasma, and whole blood.

2. The method according to claim 1, further **characterized in that** said sample is selected from serum or plasma.

3. The method according to any of the claims 1 and 2, further **characterized in that** said one or more other marker is a marker of inflammation.

4. The method according to claim 1, further **characterized in that** said one or more other marker is NT-proBNP.

5. The method according to claim 1 further **characterized in that** said one or more other marker is troponin T.

6. The method according to claim 1, wherein the marker endostatin is measured in a sample obtained from an individual at risk for heart failure.

7. Use of protein endostatin as a marker molecule in the diagnosis of heart failure in a sample selected from the group consisting of serum, plasma, and whole blood.

8. Use of a marker combination comprising endostatin and one or more other marker of heart failure in the diagnosis of heart failure, wherein the one or more other marker is selected from the group consisting of a natriuretic peptide marker, a cardiac troponin marker, and a marker of inflammation in a sample selected from the group consisting of serum, plasma, and whole blood.

9. Use of a marker combination according to claim 8 comprising at least endostatin, and NT-proBNP.

## Patentansprüche

1. Verfahren zur Diagnose von Herzinsuffizienz bei einem Individuum, welches die folgenden Schritte umfasst
a) das Messen der Konzentration des Markers Endostatin in einer von dem Individuum erhaltenen Probe,
b) gegebenenfalls das Messen der Konzentration eines oder mehrerer anderer Marker für Herzinsuffizienz in der Probe, wobei der eine oder die mehreren anderen Marker aus der aus einem natriuretischen Peptidmarker, einem kardialen Troponinmarker und einem Entzündungsmarker bestehenden Gruppe ausgewählt sind, und
c) das Diagnostizieren von Herzinsuffizienz durch Vergleichen der in Schritt (a) bestimmten Konzentration und gegebenenfalls der in Schritt (b) bestimmten Konzentration(en) mit der in einer Kontrollprobe festgestellten Konzentration dieses Markers oder dieser Marker,
wobei die Probe aus der aus Serum, Plasma und Vollblut bestehenden Gruppe ausgewählt ist.

2. Verfahren nach Anspruch 1, weiterhin **dadurch gekennzeichnet, dass** die Probe aus Serum und Plasma ausgewählt ist.

3. Verfahren nach Anspruch 1 oder 2, weiterhin **dadurch gekennzeichnet, dass** es sich bei dem einen oder den mehreren anderen Markern um einen Entzündungsmarker handelt.

4. Verfahren nach Anspruch 1, weiterhin **dadurch gekennzeichnet, dass** es sich bei dem einen oder den mehreren anderen Markern um NT-proBNP handelt.

5. Verfahren nach Anspruch 1, weiterhin **dadurch gekennzeichnet, dass** es sich bei dem einen oder den mehreren anderen Markern um Troponin T handelt.

6. Verfahren nach Anspruch 1, wobei der Marker Endostatin in einer von einem Individuum mit Herzinsuffizienzrisiko erhaltenen Probe gemessen wird.

7. Verwendung des Proteins Endostatin als Markermolekül bei der Diagnose von Herzinsuffizienz in einer aus der aus Serum, Plasma und Vollblut bestehenden Gruppe ausgewählten Probe.

8. Verwendung einer Endostatin und einen oder mehrere andere Marker von Herzinsuffizienz umfassenden Markerkombination bei der Diagnose von Herzinsuffizienz, wobei der eine oder die mehreren anderen Marker aus der aus einem natriuretischen Peptidmarker, einem kardialen Troponinmarker und einem Entzündungsmarker bestehenden Gruppe in einer aus der aus Serum, Plasma und Vollblut bestehenden Gruppe ausgewählten Probe ausgewählt sind.

9. Verwendung einer Markerkombination nach Anspruch 8, welche mindestens Endostatin und NT-proBNP umfasst.

## Revendications

1. Procédé pour le diagnostic de l'insuffisance cardiaque chez un individu comprenant les étapes de
a) mesure dans un échantillon prélevé chez l'individu, de la concentration de l'endostatine marqueur,
b) mesure éventuellement dans l'échantillon de la concentration d'un ou de plusieurs autres marqueur(s) de l'insuffisance cardiaque, dans laquelle ledit un ou plusieurs autres marqueur(s) est(sont) choisi(s) dans le groupe constitué par un marqueur peptidique natriurétique, un marqueur de la troponine cardiaque, et un marqueur de l'inflammation et
c) diagnostic de l'insuffisance cardiaque par comparaison de la concentration déterminée à l'étape (a) et éventuellement de la(des) concentration(s) déterminée(s) à l'étape (b) à la concentration de ce marqueur ou de ces marqueurs telle qu'établie dans un échantillon témoin,
dans lequel ledit échantillon est choisi dans le groupe constitué par le sérum, le plasma et le sang total.

2. Procédé selon la revendication 1, **caractérisé en outre en ce que** ledit échantillon est choisi parmi le sérum ou le plasma.

3. Procédé selon l'une quelconque des revendications 1 et 2, **caractérisé en outre en ce que** ledit un ou plusieurs autre(s) marqueur(s) est un marqueur de l'inflammation.

4. Procédé selon la revendication 1, **caractérisé en outre en ce que** ledit un ou plusieurs autre(s) marqueur(s) est le NT-proBNP.

5. Procédé selon la revendication 1, **caractérisé en outre en ce que** ledit un ou plusieurs autre(s) marqueur(s) est la troponine T.

6. Procédé selon la revendication 1, dans lequel le marqueur endostatine est mesuré dans un échantillon prélevé chez un individu à risque d'insuffisance cardiaque.

7. Utilisation de la protéine endostatine comme molécule marqueur dans le diagnostic de l'insuffisance cardiaque dans un échantillon choisi dans le groupe constitué par le sérum, le plasma et le sang total.

8. Utilisation d'une combinaison de marqueurs comprenant l'endostatine et un ou plusieurs autre(s) marqueur(s) de l'insuffisance cardiaque dans le diagnostic de l'insuffisance cardiaque, dans laquelle le ou les autres marqueurs est(sont) choisi(s) dans le groupe constitué par un marqueur peptidique natriurétique, un marqueur de la troponine cardiaque, et un marqueur de l'inflammation dans un échantillon choisi dans le groupe constitué par le sérum, le plasma et le sang total.

9. Utilisation d'une combinaison de marqueurs selon la revendication 8 comprenant au moins l'endostatine et le NT-proBNP.


### SEQUENCE LISTING

<110> Roche Diagnostics GmbH F. Hoffmann-La Roche AG
<120> Use of endostatin as a marker of heart failure
<130> 26087 WO
<150> EP09005801.7
   <151> 2009-04-27
<160> 1
<170> PatentIn version 3.3
<210> 1
   <211> 1519
   <212> PRT
   <213> Homo sapiens
<400> 1
   Met Ala Pro Tyr Pro Cys Gly Cys His Ile Leu Leu Leu Leu Phe Cys
   1               5                   10                  15
   Cys Leu Ala Ala Ala Arg Ala Asn Leu Leu Asn Leu Asn Trp Leu Trp
               20                  25                  30
   Phe Asn Asn Glu Asp Thr Ser His Ala Ala Thr Thr Ile Pro Glu Pro
           35                  40                  45
   Gln Gly Pro Leu Pro Val Gln Pro Thr Ala Asp Thr Thr Thr His Val
       50                  55                  60
   Thr Pro Arg Asn Gly Ser Thr Glu Pro Ala Thr Ala Pro Gly Ser Pro
   65                  70                  75                  80
   Glu Pro Pro Ser Glu Leu Leu Glu Asp Gly Gln Asp Thr Pro Thr Ser
                   85                  90                  95
   Ala Glu Ser Pro Asp Ala Pro Glu Glu Asn Ile Ala Gly Val Gly Ala
               100                 105                 110
   Glu Ile Leu Asn Val Ala Lys Gly Ile Arg Ser Phe Val Gln Leu Trp
           115                 120                 125
   Asn Asp Thr Val Pro Thr Glu Ser Leu Ala Arg Ala Glu Thr Leu Val
       130                 135                 140
   Leu Glu Thr Pro Val Gly Pro Leu Ala Leu Ala Gly Pro Ser Ser Thr
   145                 150                 155                 160
   Pro Gln Glu Asn Gly Thr Thr Leu Trp Pro Ser Arg Gly Ile Pro Ser
                   165                 170                 175
   Ser Pro Gly Ala His Thr Thr Glu Ala Gly Thr Leu Pro Ala Pro Thr
               180                 185                 190
   Pro Ser Pro Pro Ser Leu Gly Arg Pro Trp Ala Pro Leu Thr Gly Pro
           195                 200                 205
   Ser Val Pro Pro Pro Ser Ser Glu Arg Ile Ser Glu Glu Val Gly Leu
       210                 215                 220
   Leu Gln Leu Leu Gly Asp Pro Pro Pro Gln Gln Val Thr Gln Thr Asp
   225                 230                 235                 240
   Asp Pro Asp Val Gly Leu Ala Tyr Val Phe Gly Pro Asp Ala Asn Ser
                   245                 250                 255
   Gly Gln Val Ala Arg Tyr His Phe Pro Ser Leu Phe Phe Arg Asp Phe
               260                 265                 270
   Ser Leu Leu Phe His Ile Arg Pro Ala Thr Glu Gly Pro Gly Val Leu
           275                 280                 285
   Phe Ala Ile Thr Asp Ser Ala Gln Ala Met Val Leu Leu Gly Val Lys
       290                 295                 300
   Leu Ser Gly Val Gln Asp Gly His Gln Asp Ile Ser Leu Leu Tyr Thr
   305                 310                 315                 320
   Glu Pro Gly Ala Gly Gln Thr His Thr Ala Ala Ser Phe Arg Leu Pro
                   325                 330                 335
   Ala Phe Val Gly Gln Trp Thr His Leu Ala Leu Ser Val Ala Gly Gly
               340                 345                 350
   Phe Val Ala Leu Tyr Val Asp Cys Glu Glu Phe Gln Arg Met Pro Leu
           355                 360                 365
   Ala Arg Ser Ser Arg Gly Leu Glu Leu Glu Pro Gly Ala Gly Leu Phe
       370                 375                 380
   Val Ala Gln Ala Gly Gly Ala Asp Pro Asp Lys Phe Gln Gly Val Ile
   385 390 395 400
   Ala Glu Leu Lys Val Arg Arg Asp Pro Gln Val Ser Pro Met His Cys
   405 410 415
   Leu Asp Glu Glu Gly Asp Asp Ser Asp Gly Ala Ser Gly Asp Ser Gly
   420 425 430
   Ser Gly Leu Gly Asp Ala Arg Glu Leu Leu Arg Glu Glu Thr Gly Ala
   435 440 445
   Ala Leu Lys Pro Arg Leu Pro Ala Pro Pro Pro Val Thr Thr Pro Pro
   450 455 460
   Leu Ala Gly Gly Ser Ser Thr Glu Asp Ser Arg Ser Glu Glu Val Glu
   465 470 475 480
   Glu Gln Thr Thr Val Ala Ser Leu Gly Ala Gln Thr Leu Pro Gly Ser
   485 490 495
   Asp Ser Val Ser Thr Trp Asp Gly Ser Val Arg Thr Pro Gly Gly Arg
   500 505 510
   Val Lys Glu Gly Gly Leu Lys Gly Gln Lys Gly Glu Pro Gly Val Pro
   515 520 525
   Gly Pro Pro Gly Arg Ala Gly Pro Pro Gly Ser Pro Cys Leu Pro Gly
   530 535 540
   Pro Pro Gly Leu Pro Cys Pro Val Ser Pro Leu Gly Pro Ala Gly Pro
   545 550 555 560
   Ala Leu Gln Thr Val Pro Gly Pro Gln Gly Pro Pro Gly Pro Pro Gly
   565 570 575
   Arg Asp Gly Thr Pro Gly Arg Asp Gly Glu Pro Gly Asp Pro Gly Glu
   580 585 590
   Asp Gly Lys Pro Gly Asp Thr Gly Pro Gln Gly Phe Pro Gly Thr Pro
   595 600 605
   Gly Asp Val Gly Pro Lys Gly Asp Lys Gly Asp Pro Gly Val Gly Glu
   610 615 620
   Arg Gly Pro Pro Gly Pro Gln Gly Pro Pro Gly Pro Pro Gly Pro Ser
   625                 630                 635                 640
   Phe Arg His Asp Lys Leu Thr Phe Ile Asp Met Glu Gly Ser Gly Phe
                   645                 650                 655
   Gly Gly Asp Leu Glu Ala Leu Arg Gly Pro Arg Gly Phe Pro Gly Pro
               660                 665                 670
   Pro Gly Pro Pro Gly Val Pro Gly Leu Pro Gly Glu Pro Gly Arg Phe
           675                 680                 685
   Gly Val Asn Ser Ser Asp Val Pro Gly Pro Ala Gly Leu Pro Gly Val
       690                 695                 700
   Pro Gly Arg Glu Gly Pro Pro Gly Phe Pro Gly Leu Pro Gly Pro Pro
   705                 710                 715                 720
   Gly Pro Pro Gly Arg Glu Gly Pro Pro Gly Arg Thr Gly Gln Lys Gly
                   725                 730                 735
   Ser Leu Gly Glu Ala Gly Ala Pro Gly His Lys Gly Ser Lys Gly Ala
               740                 745                 750
   Pro Gly Pro Ala Gly Ala Arg Gly Glu Ser Gly Leu Ala Gly Ala Pro
           755                 760                 765
   Gly Pro Ala Gly Pro Pro Gly Pro Pro Gly Pro Pro Gly Pro Pro Gly
       770                 775                 780
   Pro Gly Leu Pro Ala Gly Phe Asp Asp Met Glu Gly Ser Gly Gly Pro
   785                 790                 795                 800
   Phe Trp Ser Thr Ala Arg Ser Ala Asp Gly Pro Gln Gly Pro Pro Gly
                   805                 810                 815
   Leu Pro Gly Leu Lys Gly Asp Pro Gly Val Pro Gly Leu Pro Gly Ala
               820                 825                 830
   Lys Gly Glu Val Gly Ala Asp Gly Val Pro Gly Phe Pro Gly Leu Pro
           835                 840                 845
   Gly Arg Glu Gly Ile Ala Gly Pro Gln Gly Pro Lys Gly Asp Arg Gly
   850 855 860
   Ser Arg Gly Glu Lys Gly Asp Pro Gly Lys Asp Gly Val Gly Gln Pro
   865 870 875 880
   Gly Leu Pro Gly Pro Pro Gly Pro Pro Gly Pro Val Val Tyr Val Ser
   885 890 895
   Glu Gln Asp Gly Ser Val Leu Ser Val Pro Gly Pro Glu Gly Arg Pro
   900 905 910
   Gly Phe Ala Gly Phe Pro Gly Pro Ala Gly Pro Lys Gly Asn Leu Gly
   915 920 925
   Ser Lys Gly Glu Arg Gly Ser Pro Gly Pro Lys Gly Glu Lys Gly Glu
   930 935 940
   Pro Gly Ser Ile Phe Ser Pro Asp Gly Gly Ala Leu Gly Pro Ala Gln
   945 950 955 960
   Lys Gly Ala Lys Gly Glu Pro Gly Phe Arg Gly Pro Pro Gly Pro Tyr
   965 970 975
   Gly Arg Pro Gly Tyr Lys Gly Glu Ile Gly Phe Pro Gly Arg Pro Gly
   980 985 990
   Arg Pro Gly Met Asn Gly Leu Lys Gly Glu Lys Gly Glu Pro Gly Asp
   995 1000 1005
   Ala Ser Leu Gly Phe Gly Met Arg Gly Met Pro Gly Pro Pro Gly
   1010 1015 1020
   Pro Pro Gly Pro Pro Gly Pro Pro Gly Thr Pro Val Tyr Asp Ser
   1025 1030 1035
   Asn Val Phe Ala Glu Ser Ser Arg Pro Gly Pro Pro Gly Leu Pro
   1040 1045 1050
   Gly Asn Gln Gly Pro Pro Gly Pro Lys Gly Ala Lys Gly Glu Val
   1055 1060 1065
   Gly Pro Pro Gly Pro Pro Gly Gln Phe Pro Phe Asp Phe Leu Gln
   1070 1075 1080
   Leu Glu Ala Glu Met Lys Gly Glu Lys Gly Asp Arg Gly Asp Ala
       1085                1090                1095
   Gly Gln Lys Gly Glu Arg Gly Glu Pro Gly Gly Gly Gly Phe Phe
       1100                1105                1110
   Gly Ser Ser Leu Pro Gly Pro Pro Gly Pro Pro Gly Pro Pro Gly
       1115                1120                1125
   Pro Arg Gly Tyr Pro Gly Ile Pro Gly Pro Lys Gly Glu Ser Ile
       1130                1135                1140
   Arg Gly Gln Pro Gly Pro Pro Gly Pro Gln Gly Pro Pro Gly Ile
       1145                1150                1155
   Gly Tyr Glu Gly Arg Gln Gly Pro Pro Gly Pro Pro Gly Pro Pro
       1160                1165                1170
   Gly Pro Pro Ser Phe Pro Gly Pro His Arg Gln Thr Ile Ser Val
       1175                1180                1185
   Pro Gly Pro Pro Gly Pro Pro Gly Pro Pro Gly Pro Pro Gly Thr
       1190                1195                1200
   Met Gly Ala Ser Ser Gly Val Arg Leu Trp Ala Thr Arg Gln Ala
       1205                1210                1215
   Met Leu Gly Gln Val His Glu Val Pro Glu Gly Trp Leu Ile Phe
       1220                1225                1230
   Val Ala Glu Gln Glu Glu Leu Tyr Val Arg Val Gln Asn Gly Phe
       1235                1240                1245
   Arg Lys Val Gln Leu Glu Ala Arg Thr Pro Leu Pro Arg Gly Thr
       1250                1255                1260
   Asp Asn Glu Val Ala Ala Leu Gln Pro Pro Val Val Gln Leu His
       1265                1270                1275
   Asp Ser Asn Pro Tyr Pro Arg Arg Glu His Pro His Pro Thr Ala
       1280                1285                1290
   Arg Pro Trp Arg Ala Asp Asp Ile Leu Ala Ser Pro Pro Arg Leu
       1295                1300                1305
   Pro Glu Pro Gln Pro Tyr Pro Gly Ala Pro His His Ser Ser Tyr
   1310 1315 1320
   Val His Leu Arg Pro Ala Arg Pro Thr Ser Pro Pro Ala His Ser
   1325 1330 1335
   His Arg Asp Phe Gln Pro Val Leu His Leu Val Ala Leu Asn Ser
   1340 1345 1350
   Pro Leu Ser Gly Gly Met Arg Gly Ile Arg Gly Ala Asp Phe Gln
   1355 1360 1365
   Cys Phe Gln Gln Ala Arg Ala Val Gly Leu Ala Gly Thr Phe Arg
   1370 1375 1380
   Ala Phe Leu Ser Ser Arg Leu Gln Asp Leu Tyr Ser Ile Val Arg
   1385 1390 1395
   Arg Ala Asp Arg Ala Ala Val Pro Ile Val Asn Leu Lys Asp Glu
   1400 1405 1410
   Leu Leu Phe Pro Ser Trp Glu Ala Leu Phe Ser Gly Ser Glu Gly
   1415 1420 1425
   Pro Leu Lys Pro Gly Ala Arg Ile Phe Ser Phe Asp Gly Lys Asp
   1430 1435 1440
   Val Leu Arg His Pro Thr Trp Pro Gln Lys Ser Val Trp His Gly
   1445 1450 1455
   Ser Asp Pro Asn Gly Arg Arg Leu Thr Glu Ser Tyr Cys Glu Thr
   1460 1465 1470
   Trp Arg Thr Glu Ala Pro Ser Ala Thr Gly Gln Ala Ser Ser Leu
   1475 1480 1485
   Leu Gly Gly Arg Leu Leu Gly Gln Ser Ala Ala Ser Cys His His
   1490 1495 1500
   Ala Tyr Ile Val Leu Cys Ile Glu Asn Ser Phe Met Thr Ala Ser
   1505 1510 1515
   Lys